# EUROPEAN PATENT SPECIFICATION

(11) **EP 2 101 117 B1**
(45) Date of publication and mention of the grant of the patent: **06.01.2016**
(21) Application number: 09003384.6
(22) Date of filing: 09.03.2009
(51) Int. Cl.: F24F 3/044, F24F 3/16, A61L 2/238

(54) **Air handling unit and extractor cabinets with antimicrobial surfaces**
Lüftungseinheit und Abzugsschränke mit antimikrobiellen Oberflächen
Unité de gestion de l'air et armoires d'extraction pour surfaces antimicrobiennes

(30) Priority: 10.03.2008 IT MI20080394
(43) Date of publication of application: 16.09.2009
(73) Proprietor: Librizzi, Giuseppe, 24061 Albano S. Alessandro (BG) (IT)
(72) Inventor: Librizzi, Giuseppe, 24061 Albano S. Alessandro (BG) (IT)
(74) Representative: Pandolfi, Paolo

(56) References cited:
- EP-A1- 1 647 527
- WO-A1-2007/045284
- GB-A- 2 264 165
- GB-A- 2 298 149
- GB-A- 2 300 368

## Description

### OBJECT OF THE INVENTION

The present invention relates to an air handling unit and to extractor cabinets, resolving the problem of the cleaning and disinfection of the filters and of the entire structure of said units. Said problem is resolved by means of the presence of silver ions on the internal face of the structure (usually a box-like body), and on the surfaces of all the components of said unit.

### STATE OF THE ART

The large quantities of air handled by air handling units and extractor cabinets can contain bacteria and germs. Checking, cleaning and disinfection both of the filters and of the entire structure (usually a box-like body) are essential for the healthiness of the premises served by said units.

To maintain the level of cleanliness of the air handling units, the box-like body of the structure and the filters are subjected to frequent washing, forcing a stoppage of activity in said premises. Frequent washing, however, increases the risk that it may not be possible to restore the premises to the maximum levels of hygiene necessary or specified by the legislation in force in very many countries.

### DISCLOSURE OF THE INVENTION

The present invention has as object the creation of an air handling unit which overcomes the disadvantages of known air handling units and extractor cabinets, reducing the presence of micro-organisms on the surfaces of the box-like body and of the components of said units, particularly pathogenic micro-organisms. Such a reduction makes it possible to reduce the content of micro-organisms in the air introduced into the premises served by the air handling unit or the extractor cabinet which are the subject of the present invention, to a lower level than is introduced by known air handling units, with in particular a considerable reduction in the presence of pathogenic micro-organisms. These objects are achieved by the use of silver ions applied to the inner surface of the structure of the air handling unit or extractor cabinet, usually a box-like body, and the entire surface of the components of said unit or cabinet.

An air handling unit according to the preamble of claim 1 is disclosed in GB 2 298 149 A.

The micro-organisms usually contained in normal air are destroyed by contact, thanks to the properties of said silver ions. The presence of silver ions on said surfaces of the air handling unit which is the subject of the present invention greatly reduces the percentage of micro-organisms present on said surfaces and their proliferation on them, advantageously reducing the need for frequent cleaning and disinfection processes on the unit, said silver ions being active for several years, in particular for up to ten years with the addition of zeolite to the silver ions, because said zeolite slows the release of the silver ions.

The presence of said silver ions can be achieved by applying to the entire surfaces mentioned a solution of silver ions and paint for finishing said surfaces. The minimum concentration of silver ions for obtaining an advantageous antimicrobial effect is 0.5% by weight of the solution containing silver ions applied to said surfaces.

Zeolite is added to said surfaces to slow the release of the silver ions, thus obtaining a duration for the antimicrobial action of the silver ions of years, up to ten years. Said presence of silver ions on the mentioned surfaces can also be achieved on the basis of the common knowledge of a person skilled in the art, without departing from the protective scope of the claims below.

Said surfaces are preferably made of metallic material or clad in metallic or plastic material, on which silver ions are present in the minimum concentration quoted above. Said materials are preferred because their structure responds advantageously to the function of maintaining the silver ions on the surface so that they can perform their antimicrobial function, particularly in dealing with pathogenic micro-organisms.

In the present description, frequent reference has been made to silver ions present on the internal surface of the structure of the air handling unit and on its components. It is obvious that the protective scope of the claims stated below extends equally to extractor cabinets. It is evident that the procedures through which the presence of silver ions is achieved on said surfaces or on the entire device which is the subject of the present invention may vary on the basis of the knowledge of a person skilled in the art, such as for example using sheets or films to which solutions containing silver ions have been applied, which are subsequently positioned on the aforementioned surfaces or over the entire device which is the subject of the present invention. Said procedures obviously do not depart from the protective scope of the claims stated below.

### DESCRIPTION OF THE DRAWINGS

Figure 1 shows a perspective view of the structure of the air handling unit object of the present invention, usually a box-likebody;
Figure 2 shows a perspective view of the structure and of the air handling unit components of the present invention.

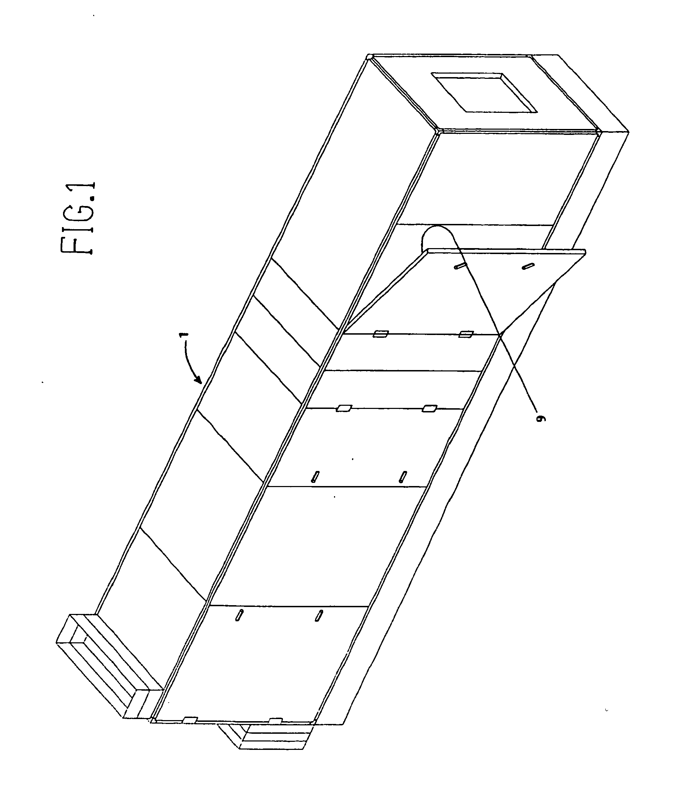

With reference to Figure 1, no. (1) indicates a structure of air handling unit object of the present invention.

In said structure (1), usually a box-like body, there are silver ions present on all the internal surfaces (9). In figure 1, by way of example, no. (9) indicates the internal surface of the floor of the structure (1), but the term 'internal surfaces' (9) is to be taken to mean all the internal surfaces of the structure (1). Said surfaces are preferably made of metallic material or clad in metallic or plastic material on which there are silver ions present. The presence of said silver ions can be achieved by applying to all the surfaces (9) a solution of silver ions and finishing paint for said surfaces (9), with a minimum, preferred, concentration of silver ions of 0.5% by weight of the solution containing said silver ions applied to said surfaces. Said solution preferably has zeolite added to it to slow the release of the above-mentioned silver ions. Said presence of silver ions on the mentioned surfaces can also be achieved on the basis of the common knowledge of a person skilled in the art, without departing from the protective scope of the claims below.

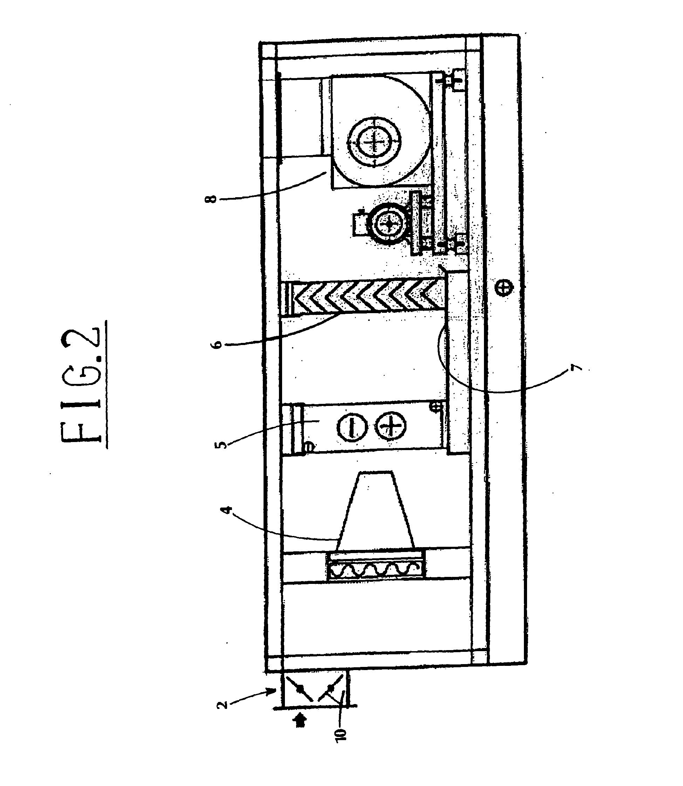

Figure 2 illustrates the components of the air handling unit which is the subject of the present invention. No. (2) indicates a shutter for an air intake of said unit. The shutter (2) provides the inner surface (10) on which the silver ions are present.

No. (3) indicates, but does not graphically represent, a recuperator, with (4) a filter, (5) a heat exchanger coil, (6) a droplet separator, (7) a condensation tray and (8) a fan.

Said components of the air handling unit which is the subject of the present invention provide silver ions over the entire surface of said components. Said surfaces on which silver ions are present are preferably made of metallic material or clad in metallic or plastic material on which there are silver ions present.

As has been explained, said materials are preferred because their structure responds advantageously to the function of maintaining the silver ions on the surface so that they can perform their antimicrobial function.

In the case of the components of the air handling unit, the presence of said silver ions can be achieved with the same procedures as were indicated in relation to the structure (1). The minimum, preferred, concentration of silver ions in this case too is 0.5% by weight of the solution containing silver ions applied to said surfaces. In the case of said components, too, said solution preferably has zeolite added to it to slow the release of the above-mentioned silver ions.

### MODE FOR CARRYING OUT THE PRESENT INVENTION

The present invention can be used for all air handling units, in particular for units serving environments in which it is necessary to have a specified air quality. Said air quality is required in environments such as operating theatres, hospitals, industries in which food products are prepared, and the semiconductor production industries.

Said applications are certainly not the only ones, seeing that the adoption of air conditioning systems for premises of various types is becoming increasingly frequent, with the consequent use of air handling units and extractor cabinets.

## Claims

1. Air handling unit comprising a structure (1), a shutter (2), at least one recuperator (3), at least one filter (4), at least one heat exchanger coil (5), at least one droplet separator (6), at least one condensation tray (7), at least one fan (8), and a layer of a solution of silver ions and finishing paint applied on internal surfaces (9) of the structure (1), **characterised in that** said solution has zeolite added to it to slow the release of said silver ions.

2. Air handling unit according to claim 1, **characterised in that** the surfaces (9) are made of metallic or plastic material.

3. Air handling unit according to claim 1, **characterised in that** on the entire internal surface (10) of the shutter (2) silver ions are present.

4. Air handling unit according to claim 1, **characterised in that** on the entire surface of each recuperator (3) silver ions are present.

5. Air handling unit according to claim 1, **characterised in that** on the entire surface of each filter (4) silver ions are present.

6. Air handling unit according to claim 1, **characterised in that** on the entire surface of each heat exchanger coil (5) silver ions are present.

7. Air handling unit according to claim 1, **characterised in that** on the entire surface of each droplet separator (6) silver ions are present.

8. Air handling unit according to claim 1, **characterised in that** on the entire surface of each condensation tray (7) silver ions are present.

9. Air handling unit according to claim 1, **characterised in that** on the entire surface of each fan (8) silver ions are present.

10. Air handling unit according to any of claims 3 to 9, **characterised in that** silver ions are present on the surface or surfaces in which silver ions are present by way of finishing paint or other means applied to said surfaces.

11. Air handling unit according to claim 1, **characterised in that** said solution has a minimum concentration of silver ions of 0,5% by weight of the solution containing said silver ions.

12. Air handling unit according to any one of claims 3 to 11, **characterised in that** the surfaces on which silver ions are present are made of metallic or plastic material.

## Patentansprüche

1. Luftbehandlungseinheit mit einer Struktur (1), einem Verschluss (2), mindestens einem Abhitzeverwerter (3), mindestens einem Filter (4), mindestens einer Wärmetauscherspule (5), mindestens einem Tröpfchenabscheider (6), mindestens einer Kondensationsschale (7), mindestens einem Gebläse (8) und einer Schicht aus einer Lösung von Silberionen und Deckanstrich, die auf innere Oberflächen (9) der Struktur (1) aufgebracht ist, **dadurch gekennzeichnet, dass** zu der Lösung Zeolith zugegeben ist, um die Freisetzung der Silberionen zu verlangsamen.

2. Luftbehandlungseinheit nach Anspruch 1, **dadurch gekennzeichnet, dass** die Oberflächen (9) aus Metall- oder Kunststoffmaterial bestehen.

3. Luftbehandlungseinheit nach Anspruch 1, **dadurch gekennzeichnet, dass** auf der ganzen inneren Oberfläche (10) des Verschlusses (2) Silberionen vorhanden sind.

4. Luftbehandlungseinheit nach Anspruch 1, **dadurch gekennzeichnet, dass** auf der ganzen Oberfläche jedes Abhitzeverwerters (3) Silberionen vorhanden sind.

5. Luftbehandlungseinheit nach Anspruch 1, **dadurch gekennzeichnet, dass** auf der ganzen Oberfläche jedes Filters (4) Silberionen vorhanden sind.

6. Luftbehandlungseinheit nach Anspruch 1, **dadurch gekennzeichnet, dass** auf der ganzen Oberfläche jeder Wärmetauscherspule (5) Silberionen vorhanden sind.

7. Luftbehandlungseinheit nach Anspruch 1, **dadurch gekennzeichnet, dass** auf der ganzen Oberfläche jedes Tröpfchenabscheiders (6) Silberionen vorhanden sind.

8. Luftbehandlungseinheit nach Anspruch 1, **dadurch gekennzeichnet, dass** auf der ganzen Oberfläche jeder Kondensationsschale (7) Silberionen vorhanden sind.

9. Luftbehandlungseinheit nach Anspruch 1, **dadurch gekennzeichnet, dass** auf der ganzen Oberfläche jedes Gebläses (8) Silberionen vorhanden sind.

10. Luftbehandlungseinheit nach einem der Ansprüche 3 bis 9, **dadurch gekennzeichnet, dass** Silberionen auf der Oberfläche oder Oberflächen vorhanden sind, in denen Silberionen durch einen Deckanstrich oder andere auf die Oberflächen aufgebrachte Mittel vorhanden sind.

11. Luftbehandlungseinheit nach Anspruch 1, **dadurch gekennzeichnet, dass** die Lösung eine minimale Konzentration von Silberionen von 0,5 Gewichts-% der Lösung, die die Silberionen enthält, aufweist.

12. Luftbehandlungseinheit nach einem der Ansprüche 3 bis 11, **dadurch gekennzeichnet, dass** die Oberflächen, auf denen Silberionen vorhanden sind, aus Metall- oder Kunststoffmaterial bestehen.

## Revendications

1. Unité de traitement d'air comprenant une structure (1), un obturateur (2), au moins un récupérateur (3), au moins un filtre (4), au moins une bobine d'échangeur de chaleur (5), au moins un séparateur de gouttelettes (6), au moins un plateau de condensation (7), au moins un ventilateur (8) et une couche d'une solution d'ions argent et d'une peinture de finition appliquée sur des surfaces internes (9) de la structure (1), **caractérisée en ce qu'**un zéolite est ajouté à ladite solution pour ralentir la libérations desdits ions argent.

2. Unité de traitement d'air selon la revendication 1, **caractérisée en ce que** les surfaces (9) sont faites d'une matière métallique ou plastique.

3. Unité de traitement d'air selon la revendication 1, **caractérisée en ce que** sur la surface interne complète (10) de l'obturateur (2), des ions argent sont présents.

4. Unité de traitement d'air selon la revendication 1, **caractérisée en ce que** sur la surface complète de chaque récupérateur (3), des ions argent sont présents.

5. Unité de traitement d'air selon la revendication 1, **caractérisée en ce que** sur la surface complète de chaque filtre (4), des ions argent sont présents.

6. Unité de traitement d'air selon la revendication 1, **caractérisée en ce que** sur la surface complète de chaque bobine d'échangeur de chaleur (5), des ions argent sont présents.

7. Unité de traitement d'air selon la revendication 1, **caractérisée en ce que** sur la surface complète de chaque séparateur de gouttelettes (6), des ions argent sont présents.

8. Unité de traitement d'air selon la revendication 1, **caractérisée en ce que** sur la surface complète de chaque plateau de condensation (7), des ions argent sont présents.

9. Unité de traitement d'air selon la revendication 1, **caractérisée en ce que** sur la surface complète de chaque ventilateur (8), des ions argent sont présents.

10. Unité de traitement d'air selon l'une quelconque des revendications 3 à 9, **caractérisée en ce que** des ions argent sont présents sur la surface ou les surfaces dans lesquelles des ions argent sont présents au moyen d'une peinture de finition ou d'autres moyens appliqués auxdites surfaces.

11. Unité de traitement d'air selon la revendication 1, **caractérisée en ce que** ladite solution a une concentration minimale en ions argent de 0,5 % en poids de la solution contenant lesdits ions argent.

12. Unité de traitement d'air selon l'une quelconque des revendications 3 à 11, **caractérisée en ce que** les surfaces sur lesquelles des ions argent sont présents sont faites d'une matière métallique ou plastique.
